# EUROPEAN PATENT APPLICATION

(11) **EP 4 838 465 A1**
(43) Date of publication of application: **11.11.2026**
(21) Application number: 26174960.0
(22) Date of filing: 28.04.2026
(51) Int. Cl.: G06T 7/33, G06T 11/00

(54) **DENTAL ALIGNMENT ON VIDEO AND MOTION**

(30) Priority: 06.05.2025 NL 2040316; 25.06.2025 NL 2040643
(71) Applicant: Smilecloud S.r.l., 300088 Timisoara, Timis County (RO)
(72) Inventor: COFAR, Florin-Nicolae, 300088 Timisoara, Timis County (RO); NICOLAE, Titus, 300088 Timisoara, Timis County (RO); SIMONIA, Mihai, 300088 Timisoara, Timis County (RO); DIACONESCU, Cristian-Florin, 300088 Timisoara, Timis County (RO); SARGHE, Radu-Florin, 300088 Timisoara, Timis County (RO)
(74) Representative: Arnold & Siedsma

(57) **Abstract**

A computer-implemented method for generating a dental design, the method comprising:
- obtaining a digital video recording of a patient's face;
- obtaining a digital three-dimensional intraoral scan of the patient's mouth, the intraoral scan comprising a digital representation of a plurality of teeth;
- processing the digital video recording to identify and separate a plurality of good frames and one or more bad frames based on predetermined criteria;
- aligning the digital three-dimensional intraoral scan to the plurality of good frames based on facial features visible in both the intraoral scan and the good frames; and
- generating a composite video using at least the plurality of good frames, wherein the aligned digital three-dimensional intraoral scan is superimposed on each of the good frames to provide a dynamic visual representation of the patient's teeth in relation to their facial appearance.

## Description

### Field of Invention

The present invention relates to the field of dental imaging and design, and more particularly to techniques for visualizing and planning dental treatments by dynamically combining facial images and three-dimensional intraoral scans.

### Background

Currently, dentists and orthodontists use a variety of methods to plan dental treatments and restorations. These methods often involve creating physical models of the patient's teeth, taking photographs of the patient's face, and using specialized software to design restorations or plan orthodontic movements. However, these traditional methods have several drawbacks.

One common approach involves creating plaster models of the patient's teeth from dental impressions. While these models provide a three-dimensional representation of the patient's dentition, they do not capture the dynamic relationship between the teeth and the patient's facial features during speech, smiling, or other expressions. This is limiting as it is known that the teeth's appearance is substantially affected by these dynamic relations. Furthermore, the fabrication of plaster models is time-consuming, requires specialized equipment and materials, and can be uncomfortable for the patient.

Another approach involves taking photographs or videos of the patient's face. While these images capture the patient's facial appearance, they do not provide accurate three-dimensional information about the teeth. This makes it difficult to precisely design dental restorations or plan orthodontic treatments that are in harmony with the patient's facial aesthetics.

Dental design software has been developed to aid in the design process, this software typically relies on static data from intraoral scans or photographs. While this software can be useful, it often lacks the ability to visualize the dynamic relationship between the teeth and the patient's facial features. This can lead to suboptimal treatment plans that do not fully consider the patient's overall facial aesthetics.

US20230132126 describes a method for generating facial lines for dental treatment planning. US 10758322 describes a method for virtually representing an orthodontic treatment outcome using automated detection facial and dental reference objects.

Therefore, there is a need for a method that allows dentists and orthodontists to visualize and plan dental treatments in a way that takes into account the dynamic relationship between the teeth and the patient's facial features.

### Summary

According to a first aspect there is provided a computer-implemented method for generating a dental design, the method comprising obtaining a digital video recording of a patient's face, obtaining a digital three-dimensional intraoral scan of the patient's mouth, the intraoral scan comprising a digital representation of a plurality of teeth, processing the digital video recording to identify and separate a plurality of good frames and one or more bad frames based on predetermined criteria, aligning the digital three-dimensional intraoral scan to the plurality of good frames based on facial features visible in both the intraoral scan and the good frames, and generating a composite video using at least the plurality of good frames, wherein the aligned digital three-dimensional intraoral scan is superimposed on each of the good frames to provide a dynamic visual representation of the patient's teeth in relation to their facial appearance.

One technical advantage of this arrangement is that it enables a more efficient use of computational resources by selectively processing only the good frames from the digital video recording. By not aligning to frames that are blurred, poorly lit, or where the patient's expression is not suitable, the system reduces the amount of data that needs to be analyzed, leading to faster processing times and reduced storage requirements. The alignment of the intraoral scan with the good frames, rather than the entire video, further optimizes processing by focusing computational effort on the most relevant and high-quality data. The superimposition of the aligned 3D scan onto the selected good frames generates a dynamic visual representation, which provides a more intuitive and informative display for the user while minimizing the bandwidth required for data transmission compared to transmitting the full 3D scan data. Furthermore, reducing the amount of data transmission also reduces the energy consumption of the device.

According to a further aspect a computer-implemented method for generating a dental design is provided, the method comprising obtaining a digital video recording of a patient's face; obtaining a digital three-dimensional intraoral scan of a patient's mouth, the intraoral scan comprising a digital representation of a plurality of teeth; processing the digital video recording to identify and separate a plurality of good frames and one or more bad frames based on predetermined criteria; aligning the digital three-dimensional intraoral scan to the plurality of good frames based on facial features visible in both the intraoral scan and the good frames, wherein aligning the digital three-dimensional intraoral scan to the plurality of good frames comprises performing an alignment on a frame-by-frame basis; weighting each of the good frames based on a confidence score derived from the accuracy of the alignment; and generating a composite video using at least the plurality of good frames, wherein the aligned digital three-dimensional intraoral scan is superimposed on each of the good frames to provide a dynamic visual representation of the patient's teeth in relation to their facial appearance, wherein generating the composite video comprises displaying frames with higher confidence scores for a longer duration than frames with lower confidence scores.

According to an exemplary embodiment, the computer-implemented method comprises processing the digital video recording to identify and separate a plurality of good frames and one or more bad frames based on the predetermined criteria comprises selecting frames where the plurality of teeth of the patient are visible as good frames. One advantage of this arrangement is that it ensures that only frames with clear visibility of the teeth are used for subsequent alignment and visualization, which improves the accuracy and reliability of the generated composite video.

According to an exemplary embodiment, the predetermined criteria comprise a sharpness and clarity of the teeth. One advantage of this arrangement is that it directly targets the visual quality of the teeth in the frames, ensuring that the selected frames are suitable for detailed analysis and design of dental restorations or orthodontic appliances.

According to an exemplary embodiment, aligning the digital three-dimensional intraoral scan to the plurality of good frames comprises performing an n-point alignment on a frame-by-frame basis. One advantage of this arrangement is that it allows for precise and dynamic alignment of the intraoral scan with the video frames.

According to an exemplary embodiment, the n-point alignment comprises identifying n corresponding anatomical landmark points on the digital three-dimensional intraoral scan and each of the good frames and calculating a transformation matrix to minimize the spatial difference between the n landmark points on the intraoral scan and the n landmark points on each good frame. One advantage of this arrangement is that it provides a robust and accurate method for aligning the 3D intraoral scan with the 2D video frames by minimizing the spatial difference between corresponding anatomical landmarks. It is noted that other alignment methods may be used, for example, an alternative alignment method comprises estimating a pose of a camera associated with each good frame relative to a digital three-dimensional intraoral scan by identifying a plurality of two-dimensional image features in the good frame, associating the two-dimensional image features with corresponding three-dimensional points on the intraoral scan, and solving a perspective-n-point optimisation to calculate a rigid transformation that minimises a reprojection error between the associated three-dimensional points projected into the good frame and their corresponding two-dimensional image features. One advantage of this arrangement is that it provides a robust and computationally efficient method for aligning the 3D intraoral scan with the 2D video frames while rejecting outlier correspondences and improving accuracy through reprojection-error minimisation.

According to an exemplary embodiment, an alternative alignment method comprises rendering, from an initial estimated pose, a projected view of the digital three-dimensional intraoral scan into a coordinate system of a good frame, extracting one or more projected contours or edges from the rendered view, extracting corresponding edges from the good frame, and iteratively updating a rigid transformation to minimise a distance metric between the projected contours or edges and the extracted edges in the good frame. One advantage of this arrangement is that it enables alignment in situations where discrete anatomical landmark points are difficult to identify, by leveraging tooth boundary geometry that is commonly visible in intraoral video.

According to an exemplary embodiment, an alternative alignment method comprises rendering a synthetic image of the digital three-dimensional intraoral scan under a selected illumination or reflectance model from a candidate pose and iteratively adjusting a rigid transformation to minimise a photometric error between the rendered synthetic image and a corresponding good frame, optionally using a robust loss function to down-weight specular highlights or occluded regions. One advantage of this arrangement is that it enables alignment using dense image information across the good frame, thereby improving stability when only a limited number of reliable correspondences are available.

According to an exemplary embodiment, an alternative alignment method comprises maximising an information-theoretic similarity measure between a rendered view of the digital three-dimensional intraoral scan and a corresponding good frame by iteratively adjusting a rigid transformation to maximise mutual information between the rendered view and the good frame. One advantage of this is that it maintains alignment performance under varying illumination, exposure, and colour response conditions that can occur during intraoral video capture.

According to an exemplary embodiment, an alternative alignment method comprises generating a three-dimensional representation from a plurality of good frames, aligning the generated three-dimensional representation to the digital three-dimensional intraoral scan using an iterative closest point procedure to calculate a rigid transformation that minimises a surface-to-surface distance, and propagating the rigid transformation to determine an alignment for one or more of the good frames. One advantage of this is that it converts a 2D-3D alignment problem into a 3D-3D alignment problem, thereby improving robustness where sufficient parallax or depth information is available.

According to an exemplary embodiment, an alternative alignment method comprises determining an initial alignment for a first good frame and tracking the alignment across subsequent good frames by estimating inter-frame motion using feature tracking or optical flow, and periodically re-estimating a rigid transformation using a global alignment procedure to reduce drift. One advantage of this is that it improves temporal stability of the alignment across a video sequence while reducing computational cost by avoiding full re-alignment for every frame.

According to an exemplary embodiment, an alternative alignment method comprises detecting one or more fiducial markers having a known spatial relationship to the dentition in each good frame and calculating a rigid transformation between the fiducial markers and the digital three-dimensional intraoral scan to align the intraoral scan to the good frame. One advantage of this is that it provides a highly repeatable and accurate alignment by using markers with known geometry, thereby reducing dependence on anatomical landmark visibility.

According to an exemplary embodiment, the transformation matrix comprises a rigid transformation including translation and rotation. One advantage of this arrangement is that it allows for accurate alignment of the intraoral scan and video frames while preserving the shape and dimensions of the teeth, resulting in a more realistic and reliable visualization.

According to an exemplary embodiment, the transformation matrix spatially registers the digital three-dimensional intraoral scan with each of the good frames, thereby aligning the digital representation of the plurality of teeth with the patient's facial appearance in the good frame. One advantage of this arrangement is that it ensures that the teeth are more accurately positioned within the patient's face in the video.

According to an exemplary embodiment, the n anatomical landmark points are selected from a group comprising: the corners of the mouth, the upper lip midline, the lower lip midline, the incisal edges of the central incisors, the canines, cusp tips, and gingival margins. One advantage of this arrangement is that it provides a diverse set of reliable and easily identifiable landmarks for accurate alignment of the intraoral scan and video frames.

According to an exemplary embodiment, the computer-implemented method further comprises weighting each of the good frames based on a confidence score derived from the accuracy of the n-point alignment. It is noted that the weighting of the good frames may be performed on a confidence scored derived from any alignment method mentioned above. One advantage of this arrangement is that it allows for prioritizing frames with more accurate alignment, further improving the reliability of the generated composite video.

According to an exemplary embodiment, generating the composite video comprises displaying frames with higher confidence scores for a longer duration than frames with lower confidence scores. One advantage of this arrangement is that it allows for focusing the viewer's attention on the most reliable and accurate frames, improving the overall quality of the visualization.

According to an exemplary embodiment, generating the composite video comprises enhancing the visual contribution of frames with higher confidence scores. One advantage of this arrangement is that it allows for improving the visual clarity of the teeth and facial features in the composite video, making it easier for dentists and orthodontists to assess the patient's condition and plan treatments.

According to an exemplary embodiment, the confidence score is inversely proportional to a root mean square (RMS) error between the n landmark points on the digital three-dimensional intraoral scan and the corresponding n landmark points on the good frame after alignment. One advantage of this arrangement is that it provides a quantitative measure of alignment accuracy, allowing for precise weighting of the good frames.

According to an exemplary embodiment, the confidence score is calculated based on a correlation coefficient between image features in the digital three-dimensional intraoral scan and corresponding image features in the good frame after alignment. One advantage of this arrangement is that it provides a robust and reliable measure of alignment accuracy, even in the presence of noise or variations in lighting.

According to an exemplary embodiment, the confidence score is determined by a machine learning model trained to predict alignment accuracy based on input features extracted from the digital three-dimensional intraoral scan and the good frame. One advantage of this arrangement is that it allows for a more sophisticated and accurate assessment of alignment quality, taking into account a variety of factors that may not be captured by simpler methods.

According to an exemplary embodiment, the computer-implemented method further comprises modifying one or more teeth in the digital three-dimensional intraoral scan to simulate a proposed dental restoration or orthodontic treatment. One advantage of this arrangement is that it allows for visualizing the potential outcomes of different treatment options, helping dentists and orthodontists make informed decisions about the best course of action.

According to an exemplary embodiment, the computer-implemented method further comprises aligning the modified digital three-dimensional intraoral scan to the plurality of good frames based on facial features visible in both the intraoral scan and the good frames and generating a modified composite video using at least the plurality of good frames, wherein the aligned modified digital three-dimensional intraoral scan is superimposed on each of the good frames to provide a dynamic visual representation of the simulated dental restoration or orthodontic treatment in relation to the patient's facial appearance. One advantage of this arrangement is that it provides a realistic and dynamic visualization of the proposed treatment outcomes, allowing dentists and orthodontists to assess the aesthetic impact and make informed decisions about the best course of action.

According to an exemplary embodiment, any one of the above described steps are applied to the modified digital three-dimensional intraoral scan and/or the modified composite video. One advantage of this arrangement is that it ensures that the modified data is processed and aligned with the same level of accuracy and reliability as the original data, improving the overall quality of the visualization.

According to an exemplary embodiment, the computer-implemented method further comprises modifying at least one teeth shown in one or more of the good frames based on the generated composite video, thereby visualizing changes to the patient's dental appearance resulting from the proposed dental restoration or orthodontic treatment. One advantage of this arrangement is that it allows for visualizing the subtle changes in the patient's facial appearance that result from the treatment, helping dentists and orthodontists to assess the overall aesthetic impact.

According to an exemplary embodiment, the computer-implemented method further comprises, based on the modified teeth in at least one good frame, modifying the one or more teeth in the remaining good frames and/or the three-dimensional intraoral scan to maintain consistency with the modification in said at least one good frame. One advantage of this arrangement is that it ensures that the changes to the teeth are consistent across the entire video and three-dimensional model, resulting in a more realistic and reliable visualization.

According to an exemplary embodiment, the generated composite video is used to design a dental restoration or orthodontic appliance. One advantage of this arrangement is that it provides a valuable tool for dentists and orthodontists to create more accurate and aesthetically pleasing restorations and appliances that are tailored to the patient's unique anatomy and facial features.

According to an exemplary embodiment, the computer-implemented method further comprises generating a dental treatment plan based on the generated composite video. One advantage of this arrangement is that it allows dentists and orthodontists to develop comprehensive and personalized treatment plans that take into account the patient's individual needs and preferences.

According to a further aspect, the computer-implemented method comprises obtaining at least one first facial image of a patient, said at least one first facial image comprising a head of the patient and a smile comprising one or more teeth, obtaining at least one first digital representation of the one or more teeth comprised by the smile, by performing a first segmentation on the obtained at least one first facial image, modifying the one or more teeth in the at least one first digital representation, applying the modified one or more teeth to the facial image of the patient, and generating a video using the modified facial image of the patient. One advantage of this arrangement is that it provides a method for generating a video of the patient with modified teeth, based on a single facial image, which can be useful for visualizing the potential outcomes of different treatment options.

According to an exemplary embodiment, generating a video comprises using a deep learning model to generate new video frames with the modified teeth integrated into the patient's facial appearance. One advantage of this arrangement is that it allows for creating a more realistic and visually appealing video, as the deep learning model can seamlessly integrate the modified teeth with the patient's facial features.

According to an exemplary embodiment, the deep learning model is a generative adversarial network GAN trained on a dataset of facial images and dental structures. One advantage of this arrangement is that it provides a state-of-the-art method for generating realistic facial images with modified teeth, as GANs are known for their ability to produce high-quality and detailed images.

According to an exemplary embodiment, the deep learning model receives input data comprising the first facial image and the modified digital representation of the teeth, and outputs a set of transformed video frames. One advantage of this arrangement is that it allows for controlling the video generation process by providing specific input data, such as the facial image and the modified teeth, which ensures that the resulting video accurately reflects the desired changes.

According to an exemplary embodiment, the deep learning model comprises a feature extraction layer that identifies key facial landmarks and dental features from the input data. One advantage of this arrangement is that it enables the model to focus on the most relevant features of the face and teeth, improving the accuracy and realism of the generated video.

According to an exemplary embodiment, the deep learning model includes a transformation layer that applies modifications to the identified facial landmarks based on the changes made to the digital representation of the teeth. One advantage of this arrangement is that it allows for creating a more natural and harmonious integration of the modified teeth with the patient's facial features, as the facial landmarks are adjusted to reflect the changes in the teeth.

According to an exemplary embodiment, the feature extraction layer utilizes a pre-trained neural network model for facial recognition tasks. One advantage of this arrangement is that it leverages the power of pre-trained models, which have been trained on large datasets of facial images, to improve the accuracy and efficiency of feature extraction.

According to an exemplary embodiment, the transformation layer incorporates a spatial transformation network to align the modifications of the teeth with the facial landmarks. One advantage of this arrangement is that it ensures that the modified teeth are accurately positioned within the patient's face, even if the patient's head is tilted or rotated.

According to an exemplary embodiment, the deep learning model further includes a temporal consistency module that maintains smooth transitions between video frames by maintaining continuity in facial expressions and modifications. One advantage of this arrangement is that it ensures that the generated video is realistic and visually appealing, as the facial expressions and modifications are consistent over time.

According to an exemplary embodiment, generating a modified video further comprises providing a sequence of facial images from a source video, transferring the modifications of the one or more teeth to the sequence of facial images, and generating new video frames based on the sequence of facial images with the transferred modifications. One advantage of this arrangement is that it allows for creating a more dynamic and realistic video, as the modifications are applied to a sequence of images rather than a single image.

According to an exemplary embodiment, the sequence of facial images from the source video is obtained from the patient. One advantage of this arrangement is that it ensures that the generated video is a true representation of the patient's own facial expressions and movements. According to an exemplary embodiment, the sequence of facial images includes frames where the patient is in different head positions. One advantage of this arrangement is that it allows for creating a more robust and versatile video, as the modifications are applied to images with different head positions, which can be useful for visualizing the treatment outcomes from different angles.

According to an exemplary embodiment, generating a second video using an unmodified version of the facial image of the patient. One advantage of this is that it provides a baseline for comparison, allowing for a clear visualization of the changes achieved by the dental design.

According to an exemplary embodiment, generating the second video comprises using a deep learning model to generate new second video frames with the unmodified version of the facial image of the patient. One advantage of this is that it ensures consistency in the video generation process, allowing for a fair comparison between the original and modified videos.

According to an exemplary embodiment, the deep learning model is a generative adversarial network GAN trained on a dataset of facial images and dental structures.

According to an exemplary embodiment, the deep learning model receives input data comprising the first facial image and an unmodified digital representation of the teeth, and outputs a set of second video frames. One advantage of this is that it allows for controlling the video generation process by providing specific input data, such as the facial image and the unmodified teeth, which ensures that the resulting video accurately reflects the original state of the patient's teeth.

According to an exemplary embodiment, the obtained at least one first facial image of the patient is used to generate the first video and the second video. One advantage of this arrangement is that it ensures that the two videos are based on the same input data, allowing for a more accurate and direct comparison of the effects of the dental design.

According to an exemplary embodiment, the computer-implemented method further comprises displaying the generated first video. One advantage of this is that it provides the user with a view of the modified teeth in the facial representation, allowing the user to visualise what the end result of the dental design would look like.

According to an exemplary embodiment, the computer-implemented method further comprises displaying the generated second video. One advantage of this is that it provides the user with a view of the original facial image prior to the application of any modifications, allowing the user to have a visual baseline with which to compare the end result.

According to an exemplary embodiment, the computer-implemented method further comprises displaying the generated first video and the generated second video. One advantage of this arrangement is that it provides a user with a side-by-side view of both the second and first video, further assisting the user in assessing the visual differences between the two scenarios.

According to an exemplary embodiment, the first video and second video are displayed side by side. One advantage of this is that this enables the user to visually compare the patient's appearance before and after the proposed dental treatment, making it easier to assess the potential benefits.

According to an exemplary embodiment, a playback of the displayed first and second video is synchronized. One advantage of this is that it allows for a more direct and intuitive comparison of the two videos, as the user can easily see how the facial expressions and movements change with the modified and original teeth.

According to an exemplary embodiment, the computer-implemented method further comprises generating a transition between the first video and the second video to gradually visualize treatment steps of the dental design. One advantage of this is that it allows for a more gradual and visually appealing presentation of the treatment steps, making it easier for the patient to understand the process and the expected outcomes.

According to an exemplary embodiment, the computer-implemented method further comprises obtaining at least one second facial image of the patient, said at least one second facial image comprising a head of the patient and a smile comprising one or more teeth after a dental treatment; and generating a third video using the at least one second facial image of the patient. One advantage of this is that it allows for incorporating real-world results into the visualization, providing a more complete and informative picture of the treatment journey.

According to an exemplary embodiment, generating the third video comprises using a deep learning model to generate video frames based on the at least one second facial image of the patient. One advantage of this is that it leverages the power of deep learning to create realistic and visually appealing videos, even from limited input data.

According to an exemplary embodiment, the third video is displayed alongside the first video and/or the second video, enabling a visual comparison of the patient's dental appearance before, during, and after the simulated dental design and/or actual dental treatment. One advantage of this is that it provides a comprehensive visual comparison of the patient's dental appearance at different stages of the treatment process, allowing for a more informed and nuanced assessment of the overall outcome.

According to an exemplary embodiment, the first video, the second video, and the third video are synchronized. One advantage of this is that it allows for a more direct and intuitive comparison of the videos, as the user can easily see how the facial expressions and movements change over time.

According to an exemplary embodiment, the computer-implemented method further comprises generating a difference map highlighting the differences between: the first video and the second video; and/or the second video and the third video; and/or the first video and the third video. One advantage of this is that it provides a quantitative measure of the changes in the patient's dental appearance, making it easier to assess the effectiveness of the treatment.

According to yet another aspect of the present invention, there is provided a computer program product comprising a computer-executable program of instructions for performing, when executed on a computer, the steps of the method of any one of the method embodiments described above.

It will be understood by the skilled person that the features and advantages disclosed hereinabove with respect to embodiments of the methods may also apply, *mutatis mutandis,* to embodiments of the computer program product.

According to yet another aspect of the present invention, there is provided a digital storage medium encoding a computer-executable program of instructions to perform, when executed on a computer, the steps of the method of any one of the method embodiments described above.

It will be understood by the skilled person that the features and advantages disclosed hereinabove with respect to embodiments of the methods may also apply, *mutatis mutandis,* to embodiments of the digital storage medium.

According to yet another aspect of the present invention, there is provided a device programmed to perform a method comprising the steps of any one of the methods of the method embodiments described above.

According to yet another aspect of the present invention, there is provided a method for downloading to a digital storage medium a computer-executable program of instructions to perform, when executed on a computer, the steps of the method of any one of the method embodiments described above.

It will be understood by the skilled person that the features and advantages disclosed hereinabove with respect to embodiments of the methods may also apply, *mutatis mutandis,* to embodiments of the method for downloading.

Further aspects of the present invention are described by the dependent claims. The features from the dependent claims, features of any of the independent claims and any features of other dependent claims may be combined as considered appropriate to the person of ordinary skill in the art, and not only in the particular combinations as defined by the claims.

### Brief description of the figures

The accompanying drawings are used to illustrate presently preferred non-limiting exemplary embodiments of devices of the present invention. The above and other advantages of the features and objects of the present invention will become more apparent and the present invention will be better understood from the following detailed description when read in conjunction with the accompanying drawings, in which:
Figure 1 schematically illustrates a flowchart of an embodiment of a computer-implemented method for generating a dental design;
Figure 2 schematically illustrates a flowchart of an exemplary embodiment of a method for aligning the 3D scan with the plurality of good frames;
Figure 3 schematically illustrates a flowchart of an embodiment of a computer-implemented method for generating a dental design.

### Description of embodiments

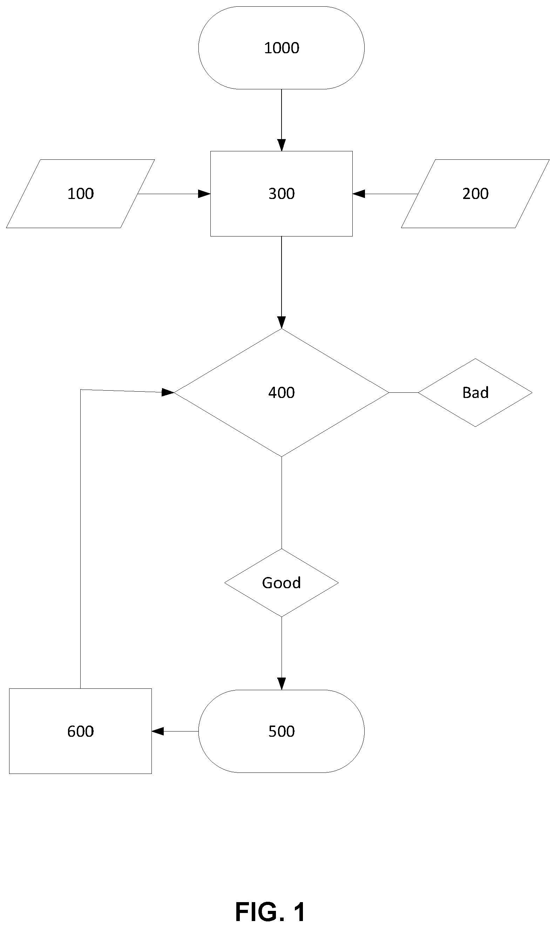

Figure 1 schematically illustrates an embodiment of a computer-implemented method 1000 for generating a dental design.

The invention can be better understood with reference to the following description. **In** the context of this application, a computer-implemented method refers to a process or series of steps that are performed by a computer or other programmable device, using software or hardware instructions. This encompasses methods that rely on computational algorithms, data processing, and automated control systems. A dental design refers to a digital representation of a dental restoration, orthodontic appliance, or other dental treatment plan that is created using computer-aided design (CAD) software. For example, the method may be used to design a crown for a damaged tooth, a bridge to replace missing teeth, or a set of aligners to straighten teeth.

It may be provided that the method comprises obtaining 100 a digital video recording of a patient's face. **In** the context of this application, obtaining a digital video recording refers to the process of capturing video data of the patient's face using a digital camera or other video recording device and storing the video data in a digital format that can be processed by a computer. This may involve using a standard digital camera, a smartphone camera, or a specialized video recording system. One advantage of this arrangement is that it provides a dynamic record of the patient's facial expressions and movements, which can be used to assess the relationship between the teeth and the face during natural movements. For example, the video may capture the patient smiling, speaking, or chewing, which can provide valuable information for treatment planning. It may be provided that a plurality of video recordings are obtained.

It may be provided that the method comprises obtaining 200 a digital three-dimensional intraoral scan of the patient's mouth, the intraoral scan comprising a digital representation of a plurality of teeth. **In** the context of this application, obtaining a digital three-dimensional intraoral scan refers to the process of capturing a three-dimensional representation of the patient's teeth and gums using an intraoral scanner. An intraoral scan refers to a digital model of the patient's mouth that is created using a handheld scanning device that is inserted into the mouth. This provides a precise and detailed three-dimensional map of the teeth and surrounding tissues. A digital representation of a plurality of teeth refers to a computer-readable file that contains the three-dimensional coordinates and surface texture of the teeth. The scan may also capture the gums and other oral structures. One advantage of this arrangement is that it provides accurate and detailed three-dimensional information about the patient's teeth and gums, which is essential for designing restorations or planning orthodontic treatments that fit precisely and function properly. For example, the intraoral scan may capture the shape, size, and position of each tooth, as well as the contours of the gums and surrounding tissues. Furthermore, the 3D scan may be obtained at various stages relative to dental treatment, including initially as a scan of the patient's mouth prior to dental treatment, during dental treatment, or even after dental treatment. This flexibility allows for comprehensive monitoring and planning throughout the dental care process, ensuring that interventions are accurately tailored to the patient's evolving oral condition.

It may be provided that the method comprises processing 300 the digital video recording to identify and separate a plurality of good frames and one or more bad frames based on predetermined criteria. **In** the context of this application, processing the digital video recording refers to the use of computer algorithms to analyze the video data and identify frames that meet certain quality standards. Predetermined criteria refers to a set of rules or guidelines that are used to evaluate the quality of the video frames. This may involve using image processing techniques to measure the sharpness, contrast, and brightness of the frames, as well as face detection algorithms to identify and exclude frames where the patient's face is not clearly visible. One advantage of this arrangement is that it improves the accuracy and efficiency of the method by eliminating frames with poor image quality or distorted facial expressions. This reduces the amount of data that needs to be processed and ensures that the alignment is based on the most reliable information. For example, predetermined criteria can be visibility of the teeth, quality of the image, or if the patient's expression is suitable.

It may be provided that the method comprises aligning 400 the digital three-dimensional intraoral scan to the plurality of good frames based on facial features visible in both the intraoral scan and the good frames. **In** the context of this application, aligning the digital three-dimensional intraoral scan refers to the process of spatially registering the 3D intraoral scan with the 2D video frames. This involves finding the best possible match between the 3D model and the 2D images, so that the teeth in the scan are accurately positioned within the patient's face in the video. Facial features visible in both the intraoral scan and the good frames refers to anatomical landmarks on the patient's face, such as the corners of the mouth, the tip of the nose, or the pupils, that can be identified in both the 3D scan and the 2D video frames. One advantage of this arrangement is that it enables the integration of the dynamic facial information with the precise dental data. This provides a more comprehensive understanding of the patient's unique anatomy and allows for the design of treatments that are in harmony with their facial features. For example, this can be achieved by identifying corresponding points on the 3D scan and the 2D images and calculating a transformation matrix that maps the 3D model onto the 2D images. To automatically identify and match facial features, several algorithms can be employed. One approach involves using feature detectors such as the Scale-Invariant Feature Transform (SIFT) or Speeded-Up Robust Features (SURF). These algorithms identify distinctive keypoints in both the 3D intraoral scan and the 2D video frames, based on local image gradients and texture patterns. These algorithms are robust to changes in scale, rotation, and illumination, making them well-suited for this application. Another approach involves using Convolutional Neural Networks (CNNs) trained for facial landmark detection. These networks can be trained to identify specific anatomical landmarks, such as the corners of the mouth, the upper lip midline, and the incisal edges of the central incisors. Once the keypoints or landmarks have been identified, matching algorithms are used to establish correspondences between the 3D scan and the 2D frames. Common matching algorithms include brute-force matching, k-nearest neighbors (KNN) matching, and random sample consensus (RANSAC). Brute-force matching simply compares all possible pairs of points and selects the closest matches based on a distance metric. KNN matching finds the 'k' nearest neighbors for each point in one set of data and selects the best match from those neighbors. RANSAC is a robust method for estimating model parameters in the presence of outliers. It iteratively selects random subsets of the data, estimates the model parameters based on those subsets, and evaluates the model on the remaining data.

A common challenge in aligning the 3D intraoral scan with the 2D video frames is that the data have different resolutions and perspectives. The 3D scan provides a high-resolution, three-dimensional representation of the teeth and gums, while the 2D video frames provide a lower-resolution, two-dimensional view of the patient's face. To address this challenge, it may be necessary to downsample the 3D scan or upsample the 2D video frames to match the resolutions. Another challenge is that the 3D scan and the 2D video frames are captured from different perspectives. The intraoral scanner is typically inserted into the patient's mouth, providing a close-up view of the teeth, while the video camera is positioned further away, providing a wider view of the face. To address this challenge, it may be necessary to use a perspective transformation to project the 3D scan onto the 2D video frames. Furthermore, since the intraoral scan does not capture the facial features, it can be provided that an additional step is to match the 3D intraoral scan to a 3D face scan. The 3D face scan is then aligned to the 2D video frames. In another embodiment, the 2D video frames are used to generate a 3D face. These methods may be combined to improve the accuracy of the resulting alignment.

It may be provided that the method comprises generating 500 a composite video using at least the plurality of good frames, wherein the aligned digital three-dimensional intraoral scan is superimposed on each of the good frames to provide a dynamic visual representation of the patient's teeth in relation to their facial appearance. In the context of this application, generating a composite video refers to the process of creating a new video sequence that combines the original video frames with the aligned three-dimensional intraoral scan. Superimposing refers to the process of overlaying the 3D model onto the 2D images, so that the teeth in the scan appear to be part of the patient's face in the video. A dynamic visual representation of the patient's teeth in relation to their facial appearance refers to a video sequence that shows the patient's teeth moving and interacting with their face in a realistic and natural way. One advantage of this arrangement is that it allows dentists and orthodontists to visualize the proposed treatment outcomes in a realistic and dynamic way. This helps them to communicate the treatment plan to the patient and to make informed decisions about the best course of action. For example, the composite video may show the patient speaking, smiling, or chewing, with the proposed dental restorations or orthodontic appliances in place. It may be provided that the composite video is generated with colors that emphasize the teeth.

It may be provided that processing 300 the digital video recording to identify and separate a plurality of good frames and one or more bad frames based on the predetermined criteria comprises selecting frames where the plurality of teeth of the patient are visible as good frames. This embodiment focuses on ensuring that the selected frames provide a clear view of the teeth for accurate alignment and visualization. Said selection may be achieved by using automated image analysis techniques to detect the presence and position of teeth in each frame. Frames where the teeth are at least partially visible, not obscured by lips or other objects, can be selected as good frames. Frames where the teeth are fully obscured, or where the lighting is poor, are classified as bad frames and discarded or processed differently. One technical advantage of this arrangement is that it significantly reduces the computational burden of the subsequent alignment and visualization steps. By focusing on frames with clear visibility of the teeth, the system avoids processing frames that are unlikely to contribute to an accurate representation of the patient's dentition. It may be provided that the frames with poor visibility are still used, but are shown with an emphasis in the visualization.

It may be provided that the predetermined criteria comprise a sharpness and clarity of the teeth. This refines the selection of good frames by focusing on image quality. Sharpness refers to the level of detail and definition in the image. Clear images exhibit high contrast and minimal blurring. These criteria may be quantified using image processing metrics such as edge detection, gradient magnitude, or frequency domain analysis. Frames that exceed a predefined threshold for sharpness and clarity are considered good frames, while frames that fall below the threshold are considered bad frames. One advantage of this arrangement is that it ensures that the selected frames are suitable for detailed analysis and accurate alignment with the intraoral scan. Sharp and clear images allow for precise identification of anatomical landmarks on the teeth, which is essential for accurate registration. For example, the assessment of sharpness can be implemented by evaluating the gradient magnitude of the image.

It may be provided that the computer-implemented method further comprises modifying 600 one or more teeth in the digital three-dimensional intraoral scan to simulate a proposed dental restoration or orthodontic treatment. **In** this context, modifying one or more teeth refers to altering the shape, size, position, or appearance of the teeth in the digital 3D intraoral scan. This is done to simulate the predicted results of a dental restoration (e.g., a crown, bridge, or veneer) or an orthodontic treatment (e.g., braces or aligners). To simulate a proposed dental restoration or orthodontic treatment means that the modifications are made in accordance with a specific treatment plan. The purpose is to visualize how the teeth will look after the treatment is completed. One advantage of this arrangement is that it allows for visualizing the potential outcomes of different treatment options, helping dentists and orthodontists make informed decisions about the best course of action. For example, the position of a tooth may be changed to simulate a treatment of braces.

It may be provided that the computer-implemented method further comprises aligning the modified digital three-dimensional intraoral scan to the plurality of good frames based on facial features visible in both the intraoral scan and the good frames; and generating a modified composite video using at least the plurality of good frames, wherein the aligned modified digital three-dimensional intraoral scan is superimposed on each of the good frames to provide a dynamic visual representation of the simulated dental restoration or orthodontic treatment in relation to the patient's facial appearance. This means that after the teeth have been digitally modified to simulate a proposed treatment, the modified 3D scan is realigned to the original video frames. The purpose is to generate a new composite video showing the patient's face with the *simulated* treatment results. One advantage of this arrangement is that it provides a realistic and dynamic visualization of the proposed treatment outcomes, allowing dentists and orthodontists to assess the aesthetic impact and make informed decisions about the best course of action. For example, the modified composite video may show how the patient's smile will look after teeth straightening or the placement of veneers.

It may be provided that any one of the steps of aligning are applied to the modified digital three-dimensional intraoral scan and/or the modified composite video. **In** this way the steps of selecting good frames, performing n-point alignment, weighting based on confidence scores, and all the related processes can be applied not only to the *original* data but also to the *modified* data after the simulation. One advantage of this arrangement is that it ensures that the modified data is processed and aligned with the same level of accuracy and reliability as the original data, improving the overall quality of the visualization. It also allows for the usage of the same algorithm across the original data and the modified data.

It may be provided that the computer-implemented method further comprises modifying at least one tooth shown in one or more of the good frames based on the generated composite video, thereby visualizing changes to the patient's dental appearance resulting from the proposed dental restoration or orthodontic treatment. This embodiment refers to directly manipulating the video frames themselves, rather than just modifying the 3D model. The generated composite video is used as a guide to alter the appearance of the teeth in the 2D video frames. One advantage of this arrangement is that it allows for visualizing the subtle changes in the patient's facial appearance that result from the treatment, helping dentists and orthodontists to assess the overall aesthetic impact. For example, the shape or color of the teeth in the video frames might be altered to reflect the proposed restoration.

It may be provided that the computer-implemented method further comprises, based on the modified teeth in at least one good frame, modifying the one or more teeth in the remaining good frames and/or the three-dimensional intraoral scan to maintain consistency with the modification in said at least one good frame. This specifies that once the operator is satisfied with the teeth shown in one or more of the good frames, the modification is then propagated across all good frames and/or the 3D intraoral scan. One advantage of this arrangement is that it ensures that the changes to the teeth are consistent across the entire video and three-dimensional model, resulting in a more realistic and reliable visualization. For example, after modifying the incisal edge of a central incisor in a particular frame, the system automatically applies the same change to the incisal edge in all other frames and in the 3D model, so that the result is consistent.

It may be provided that the generated composite video is used to design a dental restoration or orthodontic appliance. This emphasizes the practical application of the generated visualization. The composite video, with its dynamic and realistic representation of the patient's teeth in relation to their face, serves as a valuable tool for designing dental restorations or orthodontic appliances. One advantage of this arrangement is that it provides a valuable tool for dentists and orthodontists to create more accurate and aesthetically pleasing restorations and appliances that are tailored to the patient's unique anatomy and facial features. For example, the video helps to determine the optimal shape, size, and position of a crown or veneer, or to plan the precise movements of teeth during orthodontic treatment. The generated composite video can be used to design a virtual tooth.

It may be provided that the computer-implemented method further comprises generating a dental treatment plan based on the generated composite video. This highlights the ultimate goal of the method: to create a comprehensive plan for addressing the patient's dental needs. The dental treatment plan outlines the specific steps that will be taken to achieve the desired outcome, including the type of restorations or appliances to be used, the duration of treatment, and any necessary surgical procedures. One advantage of this arrangement is that it allows dentists and orthodontists to develop comprehensive and personalized treatment plans that take into account the patient's individual needs and preferences. For example, the treatment plan may specify the use of aligners to correct a malocclusion, or the placement of implants to replace missing teeth.

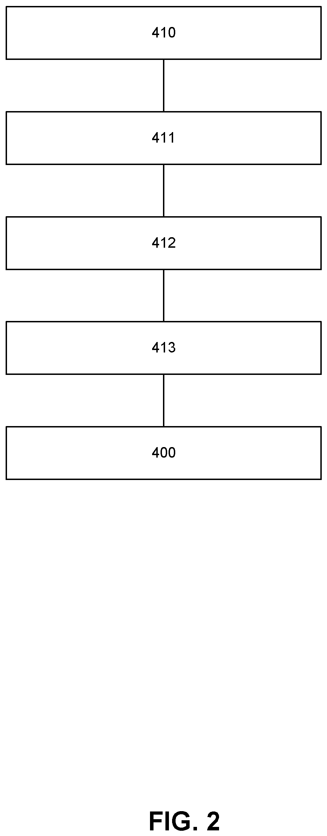

Figure 2 schematically illustrates a flowchart of an exemplary embodiment of a method for aligning the 3D scan with the plurality of good frames.

It may be provided that aligning 400 the digital three-dimensional intraoral scan to the plurality of good frames comprises performing 410 an n-point alignment on a frame-by-frame basis. **In** this context, n-point alignment refers to a technique where 'n' number of corresponding points are identified on both the 3D intraoral scan and each 2D video frame. These points serve as anchors for spatially registering the 3D model to the 2D image. Performing this alignment on a frame-by-frame basis means that the alignment process is carried out individually for *each* selected video frame. One advantage of this is that it allows the system to account for subtle changes in the patient's head position, facial expression, and lighting conditions that may occur throughout the video recording.

It may be provided that the n-point alignment 410 comprises identifying 411 n corresponding anatomical landmark points on the digital three-dimensional intraoral scan and each of the good frames, and calculating 412 a transformation matrix to minimize the spatial difference between the n landmark points on the intraoral scan and the n landmark points on each good frame. Identifying n corresponding anatomical landmark points refers to the process of manually or automatically selecting 'n' distinct points that are clearly visible and identifiable in both the 3D intraoral scan and the 2D video frames. Calculating a transformation matrix refers to the mathematical operation used to determine the optimal spatial mapping between the 3D scan and the 2D frame. The transformation matrix typically includes parameters for translation, rotation, and scaling. The transformation matrix minimizes the spatial difference between the n landmark points. One advantage of this is that it provides a robust and accurate method for aligning the 3D intraoral scan with the 2D video frames by minimizing the spatial difference between corresponding anatomical landmarks.

It may be provided that the transformation matrix comprises a rigid transformation including translation and rotation. **In** this context, a rigid transformation refers to a mathematical operation that preserves the shape and dimensions of the 3D intraoral scan while allowing it to be translated, i.e. moved and rotated in three-dimensional space. Translation refers to moving the 3D model along the X, Y, and Z axes, while rotation refers to rotating the 3D model around those axes. One advantage of this arrangement is that it allows for accurate alignment of the intraoral scan and video frames while preserving the shape and dimensions of the teeth, resulting in a more realistic and reliable visualization. For example, a rigid transformation will move and rotate, but not distort the shape of the digital three-dimensional intraoral scan.

It may be provided that the transformation matrix spatially registers 413 the digital three-dimensional intraoral scan with each of the good frames, thereby aligning the digital representation of the plurality of teeth with the patient's facial appearance in the good frame. Spatially registers refers to the process of aligning the 3D intraoral scan and 2D video frame in such a way that they are accurately positioned relative to each other in three-dimensional space. This involves transforming the 3D model to match the perspective and orientation of the 2D image. The result is that the teeth in the scan are accurately positioned within the patient's face in the video. One advantage of this arrangement is that it ensures that the teeth are accurately positioned within the patient's face in the video, providing a realistic and informative visualization for treatment planning. For example, the spatial registration allows the system to properly account for the patient's head tilt, rotation, and distance from the camera.

It may be provided that the n anatomical landmark points are selected from a group comprising: the corners of the mouth, the upper lip midline, the lower lip midline, the incisal edges of the central incisors, the canines, cusp tips, and gingival margins. Anatomical landmark points refers to easily identifiable features on the patient's face and teeth that can be used as reference points for alignment. The specific landmarks listed-corners of the mouth, upper lip midline, lower lip midline, incisal edges of the central incisors, the canines, cusp tips, and gingival margins-are all readily identifiable and relatively stable features. One advantage of this arrangement is that it provides a diverse set of reliable and easily identifiable landmarks for accurate alignment of the intraoral scan and video frames. For example, the corners of the mouth are relatively stable and easy to locate, while the incisal edges of the central incisors provide precise information about the position of the teeth.

It may be provided that the computer-implemented method further comprises weighting each of the good frames based on a confidence score derived from the accuracy of the n-point alignment. In this context, weighting each of the good frames refers to assigning a numerical value or other representative parameter to each frame, representing its reliability or accuracy in terms of alignment. A confidence score is a metric that quantifies the accuracy of the n-point alignment for a given frame. The confidence score is derived from the n-point alignment, meaning it is calculated based on the spatial relationship between the landmark points in the 3D intraoral scan and the corresponding points in the 2D video frame. One advantage of this arrangement is that it allows for prioritizing frames with more accurate alignment, further improving the reliability of the generated composite video. It may be provided that the more accurate a frame is, the higher the confidence score is.

It may be provided that generating the composite video comprises displaying frames with higher confidence scores for a longer duration than frames with lower confidence scores. In this context, displaying frames for a longer duration refers to controlling the amount of time each frame is shown in the final composite video. Frames with higher confidence scores, indicating more accurate alignment, are shown for a longer time, while frames with lower confidence scores are displayed for a shorter time. One advantage of this arrangement is that it allows for focusing the viewer's attention on the most reliable and accurate frames, improving the overall quality of the visualization. For example, if a frame is scored with a high confidence score, then the frame is displayed for 2 seconds. If it is scored with a lower one, it is only displayed for 1 second.

It may be provided that generating the composite video comprises enhancing the visual contribution of frames with higher confidence scores. In this context, enhancing the visual contribution refers to improving the visibility or prominence of the features in a frame within the composite video. This can be achieved by adjusting the brightness, contrast, sharpness, or color saturation of the frame, or by applying other image processing techniques. The amount of enhancement is proportional to the confidence score, with higher confidence frames receiving greater enhancement. One advantage of this arrangement is that it allows for improving the visual clarity of the teeth and facial features in the composite video, making it easier for dentists and orthodontists to assess the patient's condition and plan treatments. For example, a frame with the highest confidence score may be displayed with the sharpest image.

It may be provided that the confidence score is inversely proportional to a root mean square (RMS) error between the n landmark points on the digital three-dimensional intraoral scan and the corresponding n landmark points on the good frame after alignment. In this context, root mean square (RMS) error refers to a statistical measure of the difference between the predicted and actual positions of the n landmark points after alignment. The RMS error is calculated by taking the square root of the average of the squared distances between the corresponding points. Inversely proportional means that the confidence score decreases as the RMS error increases. One advantage of this arrangement is that it provides a quantitative measure of alignment accuracy, allowing for precise weighting of the good frames. For example, the confidence score may be calculated as 1/RMS error.

It may be provided that the confidence score is calculated based on a correlation coefficient between image features in the digital three-dimensional intraoral scan and corresponding image features in the good frame after alignment. In this context, a correlation coefficient refers to a statistical measure of the similarity between two sets of data. In this case, the correlation coefficient is calculated between image features (e.g., edges, corners, textures) in the 3D intraoral scan and the corresponding features in the 2D video frame. A higher correlation coefficient indicates a better match between the two sets of features, while a lower correlation coefficient indicates a poorer match. One advantage of this arrangement is that it provides a robust and reliable measure of alignment accuracy, even in the presence of noise or variations in lighting.

It may be provided that the confidence score is determined by a machine learning model trained to predict alignment accuracy based on input features extracted from the digital three-dimensional intraoral scan and the good frame. In this context, a machine learning model refers to a computer algorithm that is trained on a dataset of known inputs and outputs. In this case, the model is trained to predict alignment accuracy based on input features extracted from the 3D intraoral scan and the 2D video frame. The model uses the features to assess the quality of the alignment. One advantage of this arrangement is that it allows for a more sophisticated and accurate assessment of alignment quality, taking into account a variety of factors that may not be captured by simpler methods. For example, a machine learning model may be trained to identify and compensate for distortions in the video frames. The model processes these input features to evaluate the quality of the alignment between the 3D scan and the 2D frame. Input features may include, but are not limited to, geometric properties of the 3D scan, texture and contour data, and spatial relationships between different oral structures. The machine learning model applies its learned parameters and decision-making capabilities to predict a confidence score that reflects the alignment's accuracy.

One advantage of this approach is that it offers a more nuanced and precise assessment of alignment quality, effectively integrating multiple factors that simpler methods may overlook. The model can be trained to recognize complex distortions or anomalies in the video frames, which can impact alignment accuracy. For instance, the machine learning model may include training on variations in lighting, occlusion, or movement within the frame to ensure robust alignment predictions. The machine learning model's predictive capabilities may be achieved by employing various algorithms, such as neural networks, decision trees, or support vector machines.

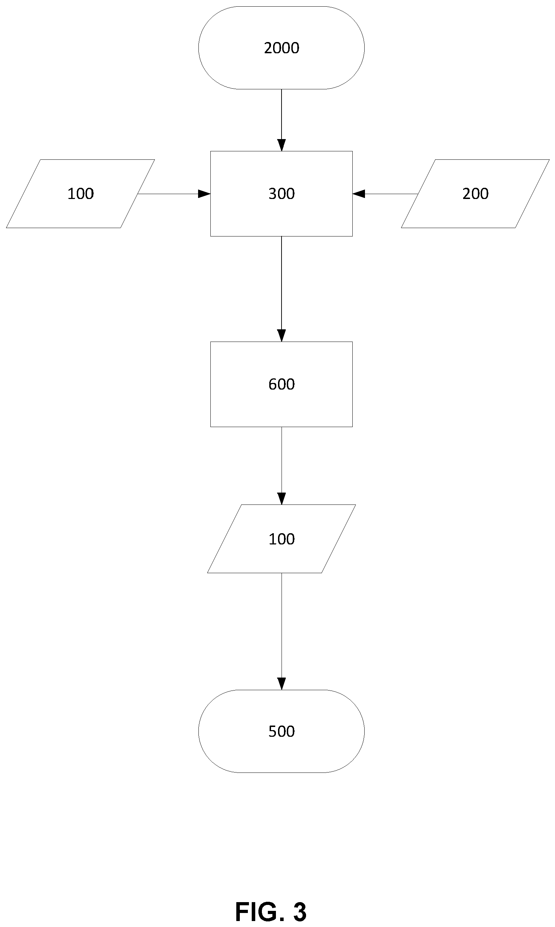

Figure 3 schematically illustrates a flowchart of an embodiment of a computer-implemented method 2000 for generating a dental design.

It may be provided that the computer-implemented method 2000 for generating a dental design comprises obtaining 100 at least one first facial image of a patient, said at least one first facial image comprising a head of the patient and a smile comprising one or more teeth; obtaining 200 at least one first digital representation of the one or more teeth comprised by the smile, by performing a first segmentation on the obtained at least one first facial image; modifying 600 the one or more teeth in the at least one first digital representation; applying the modified one or more teeth to the facial image of the patient; and generating 500 a video using the modified facial image of the patient. One advantage of this arrangement is that it provides a method for generating a video of the patient with modified teeth, based on a single facial image, which can be useful for visualizing the potential outcomes of different treatment options. In some embodiments, it may be provided that a plurality of facial images are used, and said images are combined into a single image. It may be provided that each of the steps 100, 200 and 600 comprise the same method or a combination of the methods as described above.

It may be provided that generating 500 a video comprises using a deep learning model to generate new video frames with the modified teeth integrated into the patient's facial appearance. This may also be referred to as the first video. One advantage of this arrangement is that it allows for creating a more realistic and visually appealing video, as the deep learning model can seamlessly integrate the modified teeth with the patient's facial features. For example, the deep learning model may be a generative adversarial network GAN.

It may be provided that the deep learning model is a generative adversarial network GAN trained on a dataset of facial images and dental structures. One advantage of this arrangement is that it provides a state-of-the-art method for generating realistic facial images with modified teeth, as GANs are known for their ability to produce high-quality and detailed images. For example, the GAN may be trained using a large dataset of facial images and dental structures.

It may be provided that the deep learning model receives input data comprising the first facial image and the modified digital representation of the teeth, and outputs a set of transformed video frames. One advantage of this arrangement is that it allows for controlling the video generation process by providing specific input data, such as the facial image and the modified teeth, which ensures that the resulting video accurately reflects the desired changes. For example, the input data may be compressed, or encoded.

It may be provided that the deep learning model comprises a feature extraction layer that identifies key facial landmarks and dental features from the input data. One advantage of this arrangement is that it enables the model to focus on the most relevant features of the face and teeth, improving the accuracy and realism of the generated video. For example, the feature extraction layer may use a convolutional neural network to identify key facial landmarks and dental features.

It may be provided that the deep learning model includes a transformation layer that applies modifications to the identified facial landmarks based on the changes made to the digital representation of the teeth. One advantage of this arrangement is that it allows for creating a more natural and harmonious integration of the modified teeth with the patient's facial features, as the facial landmarks are adjusted to reflect the changes in the teeth. For example, the transformation layer may use a spatial transformation network to align the modifications of the teeth with the facial landmarks.

It may be provided that the feature extraction layer utilizes a pre-trained neural network model for facial recognition tasks. One advantage of this arrangement is that it leverages the power of pre-trained models, which have been trained on large datasets of facial images, to improve the accuracy and efficiency of feature extraction. For example, the feature extraction layer may use a pre-trained VGG16 or ResNet model for facial recognition tasks.

It may be provided that the transformation layer incorporates a spatial transformation network to align the modifications of the teeth with the facial landmarks. One advantage of this arrangement is that it ensures that the modified teeth are accurately positioned within the patient's face, even if the patient's head is tilted or rotated. For example, the spatial transformation network may use a thin-plate spline transformation to align the modifications of the teeth with the facial landmarks.

It may be provided that the deep learning model further includes a temporal consistency module that maintains smooth transitions between video frames by maintaining continuity in facial expressions and modifications. One advantage of this arrangement is that it ensures that the generated video is realistic and visually appealing, as the facial expressions and modifications are consistent over time. For example, the temporal consistency module may use a recurrent neural network RNN to maintain continuity in facial expressions and modifications.

It may be provided that the transformation layer incorporates a spatial transformation network to align the modifications of the teeth with the facial landmarks. One advantage of this arrangement is that it ensures that the modified teeth are accurately positioned within the patient's face, even if the patient's head is tilted or rotated. For example, the spatial transformation network may use a thin-plate spline transformation to align the modifications of the teeth with the facial landmarks. In some embodiments, the spatial transformation network is trained to learn the optimal transformation parameters that minimize the distance between the modified teeth and the corresponding facial landmarks.

It may be provided that the deep learning model further includes a temporal consistency module that maintains smooth transitions between video frames by maintaining continuity in facial expressions and modifications. One advantage of this arrangement is that it ensures that the generated video is realistic and visually appealing, as the facial expressions and modifications are consistent over time. For example, the temporal consistency module may use a recurrent neural network RNN to maintain continuity in facial expressions and modifications. The temporal consistency module may consider multiple adjacent frames when generating each video frame to ensure continuity.

It may be provided that generating 500 a modified video further comprises providing a sequence of facial images from a source video, transferring the modifications of the one or more teeth to the sequence of facial images, and generating new video frames based on the sequence of facial images with the transferred modifications. One advantage of this arrangement is that it allows for creating a more dynamic and realistic video, as the modifications are applied to a sequence of images rather than a single image. For example, the source video may be a video of the patient speaking or smiling. The use of a source video also allows for preserving the natural dynamics of the patient's facial expressions and movements.

It may be provided that the sequence of facial images from the source video is obtained from the patient. One advantage of this arrangement is that it ensures that the generated video is a true representation of the patient's own facial expressions and movements. For example, the patient may record a short video of themselves using their smartphone. This also reduces the need for external data or models to simulate facial expressions.

It may be provided that the sequence of facial images includes frames where the patient is in different head positions. One advantage of this arrangement is that it allows for creating a more robust and versatile video, as the modifications are applied to images with different head positions, which can be useful for visualizing the treatment outcomes from different angles. For example, the sequence of facial images may include frames where the patient is looking straight ahead, to the left, and to the right.

It may be provided that generating a second video using an unmodified version of the facial image of the patient. In this context, generating a second video refers to the creation of a separate video sequence, distinct from the video showing the simulated treatment outcome, i.e. the first video. Unmodified version of the facial image means that the original facial image, prior to any digital alterations or modifications to the teeth, is used as the basis for this second video. One advantage of this is that it provides a baseline for comparison, allowing for a clear visualization of the changes achieved by the dental design, for example, the original or first video can be displayed on the left, whereas the modified video can be displayed on the right.

It may be provided that generating the second video comprises using a deep learning model to generate new second video frames with the unmodified version of the facial image of the patient. In this context, deep learning model refers to an artificial neural network that has been trained on a large dataset of facial images, and dental structures.

It may be provided that the deep learning model is a generative adversarial network GAN trained on a dataset of facial images and dental structures. In this context, a "generative adversarial network (GAN)" refers to a specific type of deep learning model that consists of two neural networks: a generator and a discriminator. The generator creates new data instances, while the discriminator evaluates their authenticity. One advantage of this arrangement is that it leverages the power of deep learning techniques to generate realistic and visually appealing videos. The GAN is trained to generate realistic facial images.

It may be provided that the deep learning model receives input data comprising the first facial image and an unmodified digital representation of the teeth, and outputs a set of second video frames. In this context, input data refers to the information that is fed into the deep learning model to generate the video frames. An unmodified digital representation of the teeth refers to the original teeth present in the facial image without any digital alterations. One advantage of this is that it allows for controlling the video generation process by providing specific input data, such as the facial image and the unmodified teeth, which ensures that the resulting video accurately reflects the original state of the patient's teeth. The input data may be an encoded representation of the features.

It may be provided that the obtained at least one first facial image of the patient is used to generate the first video and the second video. This means that the same original image(s) are used as the starting point for creating both the video with the modifications and the video without. One advantage of this is that it ensures that the two videos are based on the same input data, allowing for a more accurate and direct comparison of the effects of the dental design. For example, the same input data can be used with and without the proposed modifications to the teeth.

It may be provided that the computer-implemented method further comprises displaying the generated first video. This means that the video with the modified teeth is presented to the user. One advantage of this is that, depending on its configuration, it provides the user with the end result of the dental design, allowing the user to have a visual understanding of what the end-result will look like. For example, a video of the patient will be displayed with straight teeth, instead of the original crooked teeth.

It may be provided that the computer-implemented method further comprises displaying the generated second video. This means that the video with the original teeth - prior to the modifications - is presented to the user. One advantage of this is that, depending on its configuration, it provides the user with a visual baseline with which to compare the end result. For example, a video of the patient will be displayed with their original crooked teeth, without any modification.

It may be provided that the computer-implemented method further comprises displaying the generated first video and the generated second video. This refers to presenting both the video with the modified teeth and the video with the original teeth to the user. One advantage of this arrangement is that it provides a user with a side-by-side view of both video, further assisting the user in assessing the visual differences between the two scenarios. For example, on the left the user will see a video of the patient with their original crooked teeth, and on the right they will see a video of the patient with straight teeth.

It may be provided that the first video and second video are displayed side by side. This means that both videos are presented simultaneously, next to each other on the screen. One advantage of this is that this enables the user to visually compare the patient's appearance before and after the proposed dental treatment, making it easier to assess the potential benefits. For example, the user can easily see the differences.

It may be provided that a playback of the displayed first and second video is synchronized. This means that the two videos are played at the same time, and their playback is coordinated so that they show the same moments in time. One advantage of this is that it allows for a more direct and intuitive comparison of the two videos, as the user can easily see how the facial expressions and movements change with the modified and original teeth. For example, if the patient smiles in one video, the other video will show the patient smiling at the same time.

It may be provided that the computer-implemented method further comprises generating a transition between the first video and the second video to gradually visualize treatment steps of the dental design. This means that the system creates a smooth animation that transforms the original video into the modified video, showing the changes in the teeth in a gradual and visually appealing way. One advantage of this is that it allows for a more gradual and visually appealing presentation of the treatment steps, making it easier for the patient to understand the process and the expected outcomes. For example, the video may start with the original teeth and then gradually transform them into the proposed restoration.

It may be provided that the computer-implemented method further comprises obtaining at least one second facial image of the patient, said at least one second facial image comprising a head of the patient and a smile comprising one or more teeth after a dental treatment; and generating a third video using the at least one second facial image of the patient. In this context, "obtaining at least one second facial image" refers to the process of capturing a new facial image of the patient at a later time, specifically *after* they have undergone the planned dental treatment. This second image serves as a record of the actual, realized outcome of the treatment and enables a direct comparison with the pre-treatment baseline and the simulated results. "After a dental treatment" means that the image reflects the real-world results of the procedure, incorporating any changes made to the patient's teeth or surrounding tissues during the treatment. The image should, generally, be taken under similar conditions as the first image to help facilitate comparison. "Generating a third video" refers to creating a new video sequence based on this post-treatment image, using techniques consistent with the first and second video generation (e.g., deep learning models).

One advantage of this arrangement is that it allows for incorporating real-world results into the visualization, providing a more complete and informative picture of the treatment journey. This enables clinicians and patients to quantitatively and qualitatively assess the success of the treatment. For example, this allows the dentist to evaluate what results were achieved with the treatment, and compare these results with the original picture and the intended results for the treatment visualized in the first video. This comparison can be used to assess the effectiveness of the treatment, identify any areas for improvement, and refine future treatment plans. It also allows to quantify the results obtained, and compare them to the expected results.

It may be provided that generating the third video comprises using a deep learning model to generate video frames based on the at least one second facial image of the patient. This means that the same deep learning techniques used to create the simulated video are now applied to the real post-treatment image.

It may be provided that the third video is displayed alongside the first video and/or the second video, enabling a visual comparison of the patient's dental appearance before, during, and after the simulated dental design and/or actual dental treatment. This emphasizes a comprehensive visualization setup with three distinct points of reference: the original state, the simulated outcome, and the actual result. The "first video" represents the patient's initial dental condition before any treatment or simulation. The "second video" represents the simulated outcome of the proposed dental design, allowing for a visualization of the intended changes. The "third video" represents the actual outcome after the dental treatment has been completed.

One advantage of this is that it provides a visual comparison of the patient's dental appearance at different stages of the treatment process, allowing for a more informed and nuanced assessment of the overall outcome. This not only allows the patient to clearly visualize the possible treatment options, but also allows the healthcare provider to evaluate if the treatment goals were met. For example, if the patient did braces, the before, the intended look, and after can be compared. This side-by-side comparison allows the user to see how the actual results compare to the simulated results. It may also be provided that the videos are accompanied with key metrics that reflect the actual and intended changes.

It may be provided that the first video, the second video, and the third video are synchronized. This means that all three video streams are played back simultaneously, with their timelines aligned. One advantage of this is that it allows for a more direct and intuitive comparison of the videos, as the user can easily see how the facial expressions and movements change over time. For example, the user can compare how the patient smiles in the original, simulated, and real videos.

It may be provided that the computer-implemented method further comprises generating a difference map highlighting the differences between: the first video and the second video; and/or the second video and the third video; and/or the first video and the third video. A "difference map" refers to a visual representation that emphasizes the changes between two images or video frames. By highlighting the differences between the videos at different stages, the user can more easily identify the specific effects of the treatment. One advantage of this is that it provides a quantitative measure of the changes in the patient's dental appearance, making it easier to assess the effectiveness of the treatment. For example, one video may be shown in red and the other in blue, and the areas which overlap are white.

A person of skill in the art would readily recognize that steps of various above-described methods can be performed by programmed computers. Herein, some embodiments are also intended to cover program storage devices, e.g., digital data storage media, which are machine or computer readable and encode machine-executable or computer-executable programs of instructions, wherein said instructions perform some or all of the steps of said above-described methods. The program storage devices may be, e.g., digital memories, magnetic storage media such as a magnetic disks and magnetic tapes, hard drives, or optically readable digital data storage media. The program storage devices may be resident program storage devices or may be removable program storage devices, such as smart cards. The embodiments are also intended to cover computers programmed to perform said steps of the above-described methods.

The description and drawings merely illustrate the principles of the present invention. It will thus be appreciated that those skilled in the art will be able to devise various arrangements that, although not explicitly described or shown herein, embody the principles of the present invention and are included within its scope. Furthermore, all examples recited herein are principally intended expressly to be only for pedagogical purposes to aid the reader in understanding the principles of the present invention and the concepts contributed by the inventor(s) to furthering the art, and are to be construed as being without limitation to such specifically recited examples and conditions. Moreover, all statements herein reciting principles, aspects, and embodiments of the present invention, as well as specific examples thereof, are intended to encompass equivalents thereof.

The functions of the various elements shown in the figures, including any functional blocks labelled as processors, may be provided through the use of dedicated hardware as well as hardware capable of executing software in association with appropriate software. When provided by a processor, the functions may be provided by a single dedicated processor, by a single shared processor, or by a plurality of individual processors, some of which may be shared. Moreover, explicit use of the term processor or controller should not be construed to refer exclusively to hardware capable of executing software, and may implicitly include, without limitation, digital signal processor (DSP) hardware, network processor, application specific integrated circuit (ASIC), field programmable gate array (FPGA), read only memory (ROM) for storing software, random access memory (RAM), and non volatile storage. Other hardware, conventional and/or custom, may also be included. Similarly, any switches shown in the figures are conceptual only. Their function may be carried out through the operation of program logic, through dedicated logic, through the interaction of program control and dedicated logic, or even manually, the particular technique being selectable by the implementer as more specifically understood from the context.

It should be appreciated by those skilled in the art that any block diagrams herein represent conceptual views of illustrative circuitry embodying the principles of the present invention. Similarly, it will be appreciated that any flowcharts, flow diagrams, state transition diagrams, pseudo code, and the like represent various processes which may be substantially represented in computer readable medium and so executed by a computer.

It should be noted that the above-mentioned embodiments illustrate rather than limit the present invention and that those skilled in the art will be able to design alternative embodiments without departing from the scope of the appended claims. In the claims, any reference signs placed between parentheses shall not be construed as limiting the claim. The word comprising does not exclude the presence of elements or steps not listed in a claim. The word a or an preceding an element does not exclude the presence of a plurality of such elements. The present invention can be implemented by means of hardware comprising several distinct elements and by means of a suitably programmed computer. In claims enumerating several means, several of these means can be embodied by one and the same item of hardware. The usage of the words first, second, third, etc. does not indicate any ordering or priority. These words are to be interpreted as names used for convenience.

In the present invention, expressions such as comprise, include, have, may comprise, may include, or may have indicate existence of corresponding features but do not exclude existence of additional features.

Whilst the principles of the present invention have been set out above in connection with specific embodiments, it is to be understood that this description is merely made by way of example and not as a limitation of the scope of protection which is determined by the appended claims.

## Claims

1. A computer-implemented method (1000) for generating a dental design, the method comprising:
- obtaining (100) a digital video recording of a patient's face;
- obtaining (200) a digital three-dimensional intraoral scan of a patient's mouth, the intraoral scan comprising a digital representation of a plurality of teeth;
- processing (300) the digital video recording to identify and separate a plurality of good frames and one or more bad frames based on predetermined criteria;
- aligning (400) the digital three-dimensional intraoral scan to the plurality of good frames based on facial features visible in both the intraoral scan and the good frames, wherein aligning (400) the digital three-dimensional intraoral scan to the plurality of good frames comprises performing (410) an alignment on a frame-by-frame basis;
- weighting each of the good frames based on a confidence score derived from the accuracy of the alignment; and
- generating (500) a composite video using at least the plurality of good frames, wherein the aligned digital three-dimensional intraoral scan is superimposed on each of the good frames to provide a dynamic visual representation of the patient's teeth in relation to their facial appearance, wherein generating the composite video comprises displaying frames with higher confidence scores for a longer duration than frames with lower confidence scores.

2. The computer-implemented method according to claim 1, wherein processing (300) the digital video recording to identify and separate a plurality of good frames and one or more bad frames based on the predetermined criteria comprises selecting frames where the plurality of teeth of the patient are visible as good frames.

3. The computer-implemented method according to claim 1 or 2, wherein the predetermined criteria comprise a sharpness and clarity of the teeth.

4. The computer-implemented method any of the preceding claims, wherein aligning (400) the digital three-dimensional intraoral scan to the plurality of good frames comprises performing (410) an n-point alignment on a frame-by-frame basis.

5. The computer-implemented method according to claim 4, wherein the n-point alignment (410) comprises:
- (411) identifying n corresponding anatomical landmark points on the digital three-dimensional intraoral scan and each of the good frames; and
- (412) calculating a transformation matrix to minimize the spatial difference between the n landmark points on the intraoral scan and the n landmark points on each good frame.

6. The computer-implemented method according to claim 5, wherein the transformation matrix comprises a rigid transformation including translation and rotation; and/or wherein the transformation matrix spatially registers (413) the digital three-dimensional intraoral scan with each of the good frames, thereby aligning the digital representation of the plurality of teeth with the patient's facial appearance in the good frame.

7. The computer-implemented method according to any of the preceding claims 5-6, wherein the n anatomical landmark points are selected from a group comprising: corners of the mouth, upper lip midline, lower lip midline, incisal edges of the central incisors, canines, cusp tips, and gingival margins.

8. The computer-implemented method according to any of the preceding claims 4-7, further comprising weighting each of the good frames based on a confidence score derived from the accuracy of the n-point alignment.

9. The computer-implemented method according to any of the preceding claims, wherein generating the composite video comprises enhancing the visual contribution of frames with higher confidence scores.

10. The computer-implemented method according to any one of claims 8-9, wherein the confidence score is inversely proportional to a root mean square, RMS, error between the n landmark points on the digital three-dimensional intraoral scan and the corresponding n landmark points on the good frame after alignment.

11. The computer-implemented method according to any one of claims 8-10, wherein the confidence score is calculated based on a correlation coefficient between image features in the digital three-dimensional intraoral scan and corresponding image features in the good frame after alignment.

12. The computer-implemented method according to any one of the preceding claims, wherein the confidence score is determined by a machine learning model trained to predict alignment accuracy based on input features extracted from the digital three-dimensional intraoral scan and the good frame.

13. The computer-implemented method according to any of the preceding claims, further comprising modifying (600) one or more teeth in the digital three-dimensional intraoral scan to simulate a proposed dental restoration or orthodontic treatment.

14. The computer-implemented method according to claim 11, further comprising:
- aligning the modified digital three-dimensional intraoral scan to the plurality of good frames based on facial features visible in both the intraoral scan and the good frames; and
- generating (500) a modified composite video using at least the plurality of good frames, wherein the aligned modified digital three-dimensional intraoral scan is superimposed on each of the good frames to provide a dynamic visual representation of the simulated dental restoration or orthodontic treatment in relation to the patient's facial appearance.

15. A computer program comprising a computer-executable program of instructions for performing, when executed on a computer, the steps of the method of any one of the previous claims.
